# EUROPÄISCHE PATENTANMELDUNG

(11) **EP 3 868 719 A1**
(43) Veröffentlichungstag der Anmeldung: **25.08.2021**
(21) Anmeldenummer: 20216780.5
(22) Anmeldetag: 23.12.2020
(51) Int. Cl.: C02F 1/32, A61L 2/10

(54) **VORRICHTUNG UND SYSTEM ZUR DESINFEKTION EINES FLUIDS**

(30) Priorität: 21.02.2020 DE 202020100960 U
(71) Anmelder: WM aquatec GmbH & Co.KG, 73268 Erkenbrechtsweiler (DE)
(72) Erfinder: WÜRTEMBERGER, Michael, 73268 Erkenbrechtsweiler (DE)
(74) Vertreter: Patentanwälte Ruff, Wilhelm, Beier, Dauster & Partner mbB

(57) **Zusammenfassung**

Die Erfindung betrifft eine Vorrichtung (1) zur Desinfektion eines Fluids, vorzugsweise von Wasser, aufweisend:
- einen Fluidpfad (3), durch den das zu desinfizierende Fluid (2) befördert wird, und
- eine Strahlungsquelle (4) zur Erzeugung von Strahlung, die desinfizierend wirkt, wobei die Strahlungsquelle (4) relativ zum Fluidpfad (3) derart angeordnet ist, dass das Fluid (2) mit der Strahlung während des Förderns beaufschlag wird, wobei
- die Strahlungsquelle (4) wenigstens eine UV-LED aufweist.

Die Erfindung betrifft ferner ein System zur Desinfektion eines Fluids (2), vorzugsweise von Wasser.

## Beschreibung

Die Erfindung betrifft eine Vorrichtung sowie ein System zur Desinfektion eines Fluids, vorzugsweise von Wasser.

Vorrichtungen zur Desinfektion von Fluiden sind allgemein bekannt. Häufig weisen solche Vorrichtungen quecksilberhaltige Gasentladungslampen zur Erzeugung von UV-Strahlung auf, welche in Bezug auf die Fluide desinfizierend wirken. Nachteilig hierbei ist das Kontaminationsrisiko der zu desinfizierenden Fluide mit Quecksilber. Dies ist vor allem im Hinblick auf eine Desinfektion von Trinkwasser problematisch.

Ferner ist nachteilig, dass quecksilberhaltige Gasentladungslampen üblicherweise eine Start- oder Aufwärmphase benötigen. Dies führt zu einem erhöhten Stromverbrauch und zu einer deutlich verkürzten Lebensdauer, so dass in regelmäßigen Abständen Lampenwechsel erforderlich sind.

Aufgabe der Erfindung ist es, eine Vorrichtung zur Desinfektion eines Fluids, vorzugsweise von Wasser, sowie ein System zur Desinfektion eines Fluids, vorzugsweise von Wasser, bereitzustellen, die insbesondere die vorgenannten, im Zusammenhang von gattungsgemäßen Desinfektionsvorrichtungen erwähnten Nachteile vollständig oder teilweise umgehen.

Diese Aufgaben werden gelöst durch eine Vorrichtung zur Desinfektion eines Fluids gemäß unabhängigem Anspruch 1 sowie durch ein System zur Desinfektion eines Fluids gemäß Anspruch 15. Bevorzugte Ausgestaltungen der Vorrichtung sowie des Systems sind Gegenstand der abhängigen Ansprüche. Der Wortlaut sämtlicher Ansprüche wird hiermit durch Bezugnahme zum Inhalt der Beschreibung gemacht.

Gemäß einem ersten Aspekt betrifft die Erfindung eine Vorrichtung zur Desinfektion eines Fluids. Bei dem Fluid handelt es sich vorzugsweise um Wasser, insbesondere Trinkwasser.

Unter dem Ausdruck "Trinkwasser" soll im Sinne der vorliegenden Erfindung Wasser verstanden werden, das zum Trinken, zum Kochen, zur Zubereitung von Speisen und Getränken und/oder zur Körperpflege und -reinigung und/oder zur Reinigung von Gegenständen, die bestimmungsgemäß mit Lebensmitteln in Berührung kommen, wie beispielsweise Gläser, Geschirr und Besteck, und/oder zur Reinigung von Gegenständen, die bestimmungsgemäß nicht nur vorübergehend mit dem menschlichen Körper in Kontakt kommen, wie beispielsweise Kleidung und Wäsche, bestimmt ist.

Die Vorrichtung weist Folgendes auf:
- einen Fluidpfad, durch den das zu desinfizierende Fluid befördert wird, und
- eine Strahlungsquelle zur Erzeugung von Strahlung, die desinfizierend wirkt, wobei die Strahlungsquelle relativ zum Fluidpfad derart angeordnet ist, dass das Fluid mit der Strahlung während des Förderns oder Beförderns (des Fluids) beaufschlagt wird.

Die Vorrichtung gemäß der vorliegenden Erfindung zeichnet sich besonders dadurch aus, dass die Strahlungsquelle wenigstens eine UV-LED (Ultraviolet Light Emission Diode) aufweist oder aus wenigstens einer UV-LED (Ultraviolet Light Emission Diode) besteht.

Der Ausdruck "wenigstens eine UV-LED" kann im Sinne der vorliegenden Erfindung nur eine einzige UV-LED oder eine Mehrzahl von UV-LEDs bedeuten. Bevorzugt bedeutet der Ausdruck "wenigstens eine UV-LED" im Sinne der vorliegenden Erfindung eine Mehrzahl von UV-LEDs, d.h. zwei der mehr UV-LEDs. Dementsprechend kann die Strahlungsquelle der Vorrichtung beispielsweise zwei, drei, vier, fünf, sechs, sieben, acht, neun oder zehn UV-LEDs aufweisen.

Die Erfindung zeichnet sich insbesondere durch die folgenden Vorteile aus:
- Durch die Erfindung wird eine gefahr- oder schadstofffreie Möglichkeit zur Desinfektion von Fluiden geschaffen. So kann insbesondere durch die Verwendung wenigstens einer UV-LED anstelle von Quecksilberentladungslampen das Risiko eines Quecksilberaustritts beim Desinfizieren von Fluiden vollständig eliminiert werden. Die erfindungsgemäße Vorrichtung weist daher bevorzugt keine Gasentladungslampe, insbesondere keine quecksilberhaltige Gasentladungslampe, auf.
- Ein weiterer Vorteil der Erfindung besteht darin, dass die wenigstens eine UV-LED keine Start- oder Aufwärmphase benötigt. Die Folge ist nicht nur ein geringerer Stromverbrauch, sondern auch eine Erhöhung der Lebensdauer der Strahlungsquelle, insbesondere der wenigstens einen UV-LED, bevorzugt um mehrere Jahre, ohne dass ein Wechsel der Strahlungsquelle, insbesondere der wenigstens einen UV-LED, erforderlich ist. Die erfindungsgemäße Vorrichtung ist daher vorteilhafterweise über sehr lange Zeiträume zur Desinfektion von Fluiden einsetzbar und gleichzeitig weitgehend oder im Wesentlichen wartungsfrei.
- Ein weiterer Vorteil der Erfindung besteht darin, dass die Lebensdauer der wenigstens einen UV-LED durch Schaltzyklen (An/Aus) nicht beeinträchtigt wird. Im Gegensatz hierzu verkürzt sich die Lebensdauer herkömmlicher Gasentladungslampen, insbesondere quecksilberhaltiger Gasentladungslampen, schon bei mehreren Schaltzyklen pro Tag.
- Ein weiterer Vorteil der Erfindung besteht darin, dass die wenigstens eine UV-LED keine Wärme an das zu desinfizierende Fluid abgibt. Dadurch können Ablagerungen reduziert werden. Ferner ist hierdurch eine konstante Leistung der Strahlung (UV-Strahlung) unabhängig von der Wassertemperatur gewährleistbar.
- Ein weiterer Vorteil der Erfindung besteht darin, dass die UV-LED-Technologie eine im Gegensatz zu herkömmlichen Gasentladungslampen zuverlässige und einfach zu handhabende Technologie darstellt.
- Ferner ist von Vorteil, dass die Desinfektion des Fluids auf UV-Strahlung und mithin einem rein physikalischen Vorgang beruht. Dadurch ist der Einsatz von chemischen Desinfektionsmitteln, d.h. von desinfizierend wirkenden Chemikalien, entbehrlich. Die erfindungsgemäße Vorrichtung ist daher vorzugsweise frei von chemischen Desinfektionsmitteln.
- Ein weiterer Vorteil besteht darin, dass die der vorliegenden Erfindung zugrunde liegende UV-Desinfektion wirksam gegenüber einer Vielzahl von Krankheitserregern, wie beispielsweise Escherichia choli, Legionellen, Cryptosporidium und Giardia lamblia, ist. So kann durch die erfindungsgemäße Vorrichtung eine besonders effiziente Entkeimung von Wasser, insbesondere bis zu 99,999 %, erzielt werden.
- Ferner ist von Vorteil, dass die erfindungsgemäße Vorrichtung auf einen Fluiddurchfluss von 8 l/min bis 8,5 l/min ausgelegt sein kann, wobei noch höhere Durchflussraten durch Parallelschaltung mehrerer erfindungsgemäßer Vorrichtungen erzielbar sind. Dies ist besonders vorteilhaft im Hinblick auf eine Trinkwasserdesinfektion, beispielsweise in Reisemobilen, Expeditionsfahrzeugen, Foodtrucks oder Yachten.

In Ausgestaltung der Erfindung ist die wenigstens eine UV-LED als wenigstens eine UV-C-LED gestaltet.

Unter dem Ausdruck "UV-C-LED" soll im Sinne der vorliegenden Erfindung eine UV-LED verstanden werden, welche dazu ausgebildet ist, UV-Strahlung mit einer Wellenlänge von 100 nm bis 290 nm, insbesondere 200 nm bis 290 nm, vorzugsweise 240 nm bis 290 nm, zu erzeugen, insbesondere wenn die UV-LED aktiv ist.

Der Ausdruck "wenigstens eine UV-C-LED" kann im Sinne der vorliegenden Erfindung nur eine einzige UV-C-LED oder eine Mehrzahl von UV-C-LEDs bedeuten. Bevorzugt bedeutet der Ausdruck "wenigstens eine UV-C-LED" im Sinne der vorliegenden Erfindung eine Mehrzahl von UV-C-LEDs, d.h. zwei der mehr UV-C-LEDs. Dementsprechend kann die Strahlungsquelle der Vorrichtung beispielsweise zwei, drei, vier, fünf, sechs, sieben, acht, neun oder zehn UV-C-LEDs aufweisen.

In weiterer Ausgestaltung der Erfindung ist die wenigstens eine UV-LED, vorzugsweise die wenigstens eine UV-C-LED, dazu ausgebildet, UV-Strahlung mit einer Wellenlänge von 240 nm bis 290 nm zu erzeugen, insbesondere wenn die wenigstens eine UV-LED, vorzugsweise die wenigstens eine UV-C-LED, aktiv ist. Hierdurch ist vorteilhafterweise eine besonders effiziente Fluidentkeimung, vorzugsweise Wasserentkeimung, erzielbar.

In weiterer Ausgestaltung der Erfindung ist der Fluidpfad, insbesondere abschnittsweise, durch ein Reaktorgehäuse geführt, wobei die Strahlungsquelle in dem Reaktorgehäuse angeordnet ist.

Der Fluidpfad ist vorzugsweise schlauchförmig, insbesondere als Kunststoffschlauch, vorzugsweise als transparenter Kunststoffschlauch, gestaltet.

In weiterer Ausgestaltung der Erfindung weist die Vorrichtung ferner eine Steuereinheit auf, die dazu ausgebildet ist, einen Betrieb der Vorrichtung zu steuern, wobei die Vorrichtung einen mit der Steuereinheit datenverbundenen oder wirkverbundenen Pumpenzustands-Eingang aufweist, der mit einem Signal beaufschlagbar ist, das anzeigt, ob eine Pumpe ein Fördern oder Befördern des Fluids bewirkt, wobei die Steuereinheit dazu ausgebildet ist, die Strahlungsquelle, insbesondere die wenigstens eine UV-LED, zu aktivieren, wenn das Signal anzeigt, dass die Pumpe ein Fördern oder Befördern des Fluids bewirkt. Dadurch ist in vorteilhafter Weise ein Echtzeit-Betrieb der erfindungsgemäßen Vorrichtung möglich, d.h. eine Desinfektion des Fluids findet nur statt, wenn die Pumpe ein Fördern oder Befördern des Fluids bewirkt. Dies wiederum trägt zu einer signifikanten Erhöhung der Lebensdauer Strahlungsquelle, insbesondere der wenigstens einen UV-LED, bei. Beispielsweise ist hierdurch ein Einsatz der Strahlungsquelle, insbesondere der wenigstens einen UV-LED, und mithin der erfindungsgemäßen Vorrichtung über mehrere Jahre, insbesondere über 13 Jahre, bei vorzugsweise unbegrenzten Schaltzyklen realisierbar. Ein weiterer Vorteil des Echtzeit-Betriebs besteht in einem deutlich geringeren Stromverbrauch. Bei dem in diesem Absatz genannten Signal handelt es sich vorzugsweise um ein elektrisches Signal, insbesondere um eine Stromstärke und/oder Spannung. Bei der vorgenannten Steuereinheit handelt es sich vorzugsweise um eine elektrische Steuereinheit.

In weiterer Ausgestaltung der Erfindung weist die Vorrichtung ferner eine Temperaturüberwachungseinrichtung auf, die dazu ausgebildet ist, eine Temperatur der Strahlungsquelle, insbesondere der wenigstens einen UV-LED, zu überwachen, bevorzugt während die Strahlungsquelle, insbesondere die wenigstens eine UV-LED, aktiv ist. Bei der Temperaturüberwachungseinrichtung handelt es sich bevorzugt um eine elektrische Temperaturüberwachungseinrichtung. Vorzugsweise ist die Temperaturüberwachungseinrichtung im Reaktorgehäuse angeordnet.

In weiterer Ausgestaltung der Erfindung ist die Temperaturüberwachungseinrichtung ferner dazu ausgebildet, die Auslösung eines Alarm- oder Warnsignals, insbesondere akustischen und/oder optischen Alarm- oder Warnsignals, und/oder die Abschaltung der Vorrichtung zu bewirken, wenn die Temperatur der Strahlungsquelle, insbesondere der wenigstens einen UV-LED, einen Temperaturschwellenwertbereich von 52 °C bis 57 °C, insbesondere einen Temperaturschwellenwert von 52 °C, 53 °C, 54 °C, 55 °C, 56 °C oder 57 °C, überschreitet. Dadurch kann vorteilhafterweise eine Überhitzung der Strahlungsquelle, insbesondere der wenigstens einen UV-LED, verhindert werden. Dies wirkt sich ebenfalls positiv auf die Lebensdauer der Strahlungsquelle, insbesondere der wenigstens einen UV-LED, aus. Zur Auslösung des Alarm- oder Warnsignals kann die Vorrichtung ferner eine, insbesondere mit der Temperaturüberwachungseinrichtung daten- oder wirkverbundene, Signalgebungseinrichtung, beispielsweise in Form einer Signalhupe, aufweisen. Bevorzugt ist die Temperaturüberwachungseinrichtung ferner dazu ausgebildet, eine Aufhebung des Alarm- oder Warnsignals und/oder eine Inbetriebnahme, insbesondere Wiederinbetriebnahme, der Vorrichtung zu bewirken, wenn die Temperatur der Strahlungsquelle, insbesondere der wenigstens einen UV-LED, den vorgenannten Temperaturschwellenwertbereich oder einen der vorgenannten Temperaturschwellenwerte wieder erreicht oder unterschreitet.

Alternativ oder in Kombination kann die Vorrichtung ferner eine Temperaturüberwachungseinrichtung aufweisen, die dazu ausgebildet ist, eine Temperatur des zu desinfizierenden Fluids zu überwachen. Bei der Temperaturüberwachungseinrichtung handelt es sich bevorzugt (ebenfalls) um eine elektrische Temperaturüberwachungseinrichtung.Die die Temperaturüberwachungseinrichtung kann ferner dazu ausgebildet sein, die Auslösung eines Alarm- oder Warnsignals, insbesondere akustischen und/oder optischen Alarm- oder Warnsignals, und/oder die Abschaltung der Vorrichtung zu bewirken, wenn die Temperatur des zu desinfizierenden Fluids einen Temperaturschwellenwertbereich von 37 °C bis 42 °C, insbesondere einen Temperaturschwellenwert von 37 °C, 38 °C, 39 °C, 40 °C, 41 °C oder 42 °C, überschreitet. Dadurch kann vorteilhafterweise eine Überhitzung der Vorrichtung und mithin der Strahlungsquelle, insbesondere der wenigstens einen UV-LED, verhindert werden. Dies wirkt sich ebenfalls positiv auf die Lebensdauer der Strahlungsquelle, insbesondere der wenigstens einen UV-LED, aus. Zur Auslösung des Alarm- oder Warnsignals kann die Vorrichtung ferner eine, insbesondere mit der Temperaturüberwachungseinrichtung daten- oder wirkverbundene, Signalgebungseinrichtung, beispielsweise in Form einer Signalhupe, aufweisen. Bevorzugt ist die Temperaturüberwachungseinrichtung ferner dazu ausgebildet, eine Aufhebung des Alarm- oder Warnsignals und/oder eine Inbetriebnahme, insbesondere Wiederinbetriebnahme, der Vorrichtung zu bewirken, wenn die Temperatur des zu desinfizierenden Fluids den vorgenannten Temperaturschwellenwertbereich oder einen der vorgenannten Temperaturschwellenwerte wieder erreicht oder unterschreitet. Vorzugsweise ist die Temperaturüberwachungseinrichtung im Reaktorgehäuse angeordnet.

In weiterer Ausgestaltung der Erfindung weist die Vorrichtung ferner eine Strahlungsintensitätserfassungseinrichtung auf, die dazu ausgebildet ist, eine Intensität der Strahlung, insbesondere UV-Strahlung, zu erfassen oder zu überwachen, während die Strahlungsquelle, insbesondere die wenigstens eine UV-LED, aktiv ist. Dadurch ist in vorteilhafter Weise eine Erfassung oder Überwachung, insbesondere Echtzeiterfassung oder -überwachung, der Desinfektionsleistung der Strahlungsquelle, insbesondere der wenigstens einen UV-LED, erzielbar. Insbesondere ist hierdurch eine Überwachung der Lebensdauer und/oder eine Vorhersage der noch verbleibenden Lebensdauer der Strahlungsquelle, insbesondere der wenigstens einen UV-LED, möglich. Bei der Strahlungsintensitätserfassungseinrichtung handelt es sich bevorzugt um eine elektrische Strahlungsintensitätserfassungseinrichtung. Vorzugsweise ist die Strahlungsintensitätserfassungseinrichtung im Reaktorgehäuse angeordnet. Bei der zu erfassenden Strahlung handelt es sich bevorzugt um eine an einer Innenwand des Reaktorgehäuses reflektierte Strahlung, insbesondere UV-Strahlung. Ein weiterer Vorteil der Strahlungsintensitätserfassungseinrichtung kann darin bestehen, dass beispielsweise ein Verschmutzungsgrad des Fluids und/oder die Ausbildung von Kalkablagerungen im Reaktorgehäuse und/oder die Ausbildung eines Biofilms im Reaktorgehäuse erfasst oder bestimmt werden kann.

Vorzugsweise ist die Strahlungsintensitätserfassungseinrichtung als UV-Intensitätssensor gestaltet.

In weiterer Ausgestaltung der Erfindung ist die Strahlungsintensitätserfassungseinrichtung ferner dazu ausgebildet, die Auslösung eines Alarm- oder Warnsignals, insbesondere akustischen und/oder optischen Alarm- oder Warnsignals, und/oder die Abschaltung der Vorrichtung zu bewirken, wenn die Intensität der Strahlung, insbesondere UV-Strahlung, einen Strahlungsintensitätsschwellenwert, insbesondere einen Strahlungsintensitätsschwellenwert, der 70% der Anfangsstrahlungsintensität entspricht (sogenannter L-70-Wert), unterschreitet. Dadurch kann vorteilhafterweise gewährleistet werden, dass das zu desinfizierende Fluid einer unter Desinfektionsgesichtspunkten ausreichenden Strahlungsintensität ausgesetzt wird. Zur Auslösung des Alarm- oder Warnsignals kann die Vorrichtung ferner eine, insbesondere mit der Strahlungsintensitätserfassungseinrichtung daten- oder wirkverbundene, Signalgebungseinrichtung, beispielsweise in Form einer Signalhupe, aufweisen. Bevorzugt ist die Strahlungsintensitätserfassungseinrichtung ferner dazu ausgebildet, eine Aufhebung des Alarm- oder Warnsignals und/oder eine Inbetriebnahme, insbesondere Wiederinbetriebnahme, der Vorrichtung zu bewirken, wenn die Strahlung, insbesondere UV-Strahlung, der Strahlungsquelle, insbesondere der wenigstens einen UV-LED, den Strahlungsintensitätsschwellenwert wieder erreicht oder überschreitet.

In weiterer Ausgestaltung der Erfindung weist die Vorrichtung ferner eine Funktionsüberwachungseinrichtung auf, die dazu ausgebildet ist, eine Funktion oder einen Betrieb der Strahlungsquelle, insbesondere der wenigstens einen UV-LED, zu überwachen, bevorzugt während die Strahlungsquelle, insbesondere die wenigstens eine UV-LED, aktiv ist. Bei der Funktionsüberwachungseinrichtung handelt es sich bevorzugt um eine elektrische Funktionsüberwachungseinrichtung. Vorzugsweise ist die Strahlungsintensitätserfassungseinrichtung im Reaktorgehäuse angeordnet. In weiterer Ausgestaltung der Erfindung ist die Funktionsüberwachungseinrichtung dazu ausgebildet, eine Stromversorgung der Strahlungsquelle, insbesondere der wenigstens einen UV-LED, zu überwachen, bevorzugt während die Strahlungsquelle, insbesondere die wenigstens eine UV-LED, aktiv ist. Vorzugsweise ist die Funktionsüberwachungseinrichtung im Reaktorgehäuse angeordnet.

In weiterer Ausgestaltung der Erfindung ist die Funktionsüberwachungseinrichtung ferner dazu ausgebildet, die Auslösung eines Alarm- oder Warnsignals, insbesondere akustischen und/oder optischen Alarm- oder Warnsignals, und/oder die Abschaltung der Vorrichtung zu bewirken, wenn eine Spannung, die beim/im Betrieb der Vorrichtung die Strahlungsquelle, insbesondere die wenigstens eine UV-LED, versorgt, einen unteren Spannungsschwellenwert, insbesondere einen unteren Spannungsschwellenwert von 0,17 V oder 7 V, unterschreitet und/oder einen oberen Spannungsschwellenwert, insbesondere einen oberen Spannungsschwellenwert von 0,23 V oder 13 V, überschreitet. Hierdurch lässt sich in vorteilhafter Weise ebenfalls die Lebensdauer der Strahlungsquelle, insbesondere der wenigstens einen UV-LED, erhöhen. Zur Auslösung des Alarm- oder Warnsignals kann die Vorrichtung ferner eine, insbesondere mit der Funktionsüberwachungseinrichtung daten- oder wirkverbundene, Signalgebungseinrichtung, beispielsweise in Form einer Signalhupe, aufweisen. Bevorzugt ist die Funktionsüberwachungseinrichtung ferner dazu ausgebildet, eine Aufhebung des Alarm- oder Warnsignals und/oder eine Inbetriebnahme, insbesondere Wiederinbetriebnahme, der Vorrichtung zu bewirken, wenn die Spannung, die beim/im Betrieb der Vorrichtung die Strahlungsquelle, insbesondere die wenigstens eine UV-LED, versorgt, einen Wert erreicht, der wieder dem unteren Spannungsschwellenwert, dem oberen Spannungsschwellenwert oder einem Wert entspricht, der zwischen dem unteren Spannungsschwellenwert und dem oberen Spannungsschwellenwert liegt.

In weiterer Ausgestaltung der Erfindung weist die Vorrichtung ferner eine Betriebsüberwachungseinrichtung auf, die dazu ausgebildet ist, zu überprüfen, ob die Vorrichtung mit einer Betriebsspannung versorgt ist. Ferner weist die Vorrichtung einen mit der Betriebsüberwachungseinrichtung datenverbundenen oder wirkverbundenen Pumpenzustands-Eingang auf, der mit einem Signal beaufschlagbar ist, das anzeigt, ob eine Pumpe ein Fördern oder Befördern des Fluids bewirkt. Vorzugsweise ist die Betriebsüberwachungseinrichtung dazu ausgebildet, die Auslösung eines Alarm- oder Warnsignals, insbesondere akustischen und/oder optischen Alarm- oder Warnsignals, zu bewirken, wenn das Signal anzeigt, dass die Pumpe ein Fördern oder Befördern des Fluids bewirkt, und die Vorrichtung nicht mit einer Betriebsspannung versorgt ist. Zur Auslösung des Alarm- oder Warnsignals kann die Vorrichtung ferner eine, insbesondere mit der Betriebsüberwachungseinrichtung daten- oder wirkverbundene, Signalgebungseinrichtung, beispielsweise in Form einer Signalhupe, aufweisen. Dadurch kann mit besonderem Vorteil verhindert werden, dass das Fluid durch den Fluidpfad der Vorrichtung befördert wird, ohne dass eine Desinfektion des Fluids erfolgt. Bei der Betriebsüberwachungseinrichtung handelt es sich bevorzugt um eine elektrische Betriebsüberwachungseinrichtung.

In weiterer Ausgestaltung der Erfindung weist die Vorrichtung ferner eine Druckerfassungseinrichtung auf, die dazu ausgebildet ist, einen Druck zu erfassen, mit dem das Fluid durch den Fluidpfad der Vorrichtung befördert wird. Vorzugsweise ist die Druckerfassungseinrichtung ferner dazu ausgebildet, eine Aktivierung der Strahlungsquelle, insbesondere der wenigstens einen UV-LED, zu bewirken, wenn der Druck, mit dem das Fluid durch den Fluidpfad befördert wird, einen Druckschwellenwert unterschreitet, und/oder dazu ausgebildet, eine Deaktivierung oder Abschaltung der Strahlungsquelle zu bewirken, wenn der Druck, mit dem das Fluid durch den Fluidpfad befördert wird, einen Druckschwellenwert überschreitet. Bei der Druckerfassungseinrichtung kann es sich um eine mechanische oder elektrische Druckerfassungseinrichtung handeln. Die Druckerfassungseinrichtung ist vorzugsweise als Drucksensor gestaltet.

In weiterer Ausgestaltung der Erfindung weist die Vorrichtung ferner eine Durchflussüberwachungseinrichtung auf, die dazu ausgebildet ist, zu überprüfen, ob das Fluid durch den Fluidpfad der Vorrichtung befördert wird. Bei der Durchflussüberwachungseinrichtung kann es sich um eine elektrische oder mechanische Durchflussüberwachungseinrichtung handeln. Die Durchflussüberwachungseinrichtung ist vorzugsweise als Durchflusssensor gestaltet.

Die beiden vorherigen Ausgestaltungen der Erfindung haben insbesondere den Vorteil, dass, beispielsweise im Haus- oder Gebäudebau, gewährleistet werden kann, dass eine Desinfektion des Fluids nur beim Öffnen einer Armatur, insbesondere Wasserarmatur, stattfindet.

Die Vorrichtung kann beispielsweise nachfolgende Abmessungen aufweisen:
- eine Breite von 50 mm bis 250 mm, insbesondere 80 mm bis 200 mm, bevorzugt 100 mm bis 150 mm,
- eine Höhe von 60 mm bis 300 mm, insbesondere 100 mm bis 250 mm, bevorzugt 140 mm bis 200 mm, und
- eine Tiefe von 40 mm bis 200 mm, insbesondere 60 mm bis 150 mm, bevorzugt 80 mm bis 120 mm.

Beispielsweise kann die Vorrichtung eine Breite von 113 mm, eine Höhe von 159 mm und eine Tiefe von 105 mm aufweisen.

Vorzugsweise weist die Vorrichtung eine Masse von 0,5 kg bis 2 kg, insbesondere 0,6 kg bis 1,5 kg, bevorzugt 0,8 kg bis 1 kg, auf.

Die in den beiden vorherigen Absätzen offenbarten Ausgestaltungen der Erfindung haben den Vorteil, dass sich hierdurch insbesondere eine kompakte und/oder mobile und/oder portable Vorrichtung realisieren lässt, welche insbesondere einfach in bestehende Fluidsysteme, bevorzugt Trinkwassersysteme, nachrüstbar ist.

Die Vorrichtung weist ferner bevorzugt ein Gehäuse, insbesondere aus Edelstahl, auf, in dem die Strahlungsquelle sowie optional weitere Komponenten oder Bauteile, insbesondere die vorgenannte Steuereinheit und/oder die vorgenannte Temperaturüberwachungseinrichtung und/oder die vorgenannte Strahlungsintensitätserfassungseinrichtung und/oder die vorgenannte Funktionsüberwachungseinrichtung und/oder die vorgenannte(n) Signalgebungseinrichtung(en), angeordnet oder eingehaust sind.

Gemäß einem zweiten Aspekt betrifft die Erfindung ein System zur Desinfektion eines Fluids, vorzugsweise von Wasser, insbesondere Trinkwasser.

Das System weist eine Vorrichtung gemäß erstem Erfindungsaspekt auf.

Zusätzlich weist das System eine Druckerfassungseinrichtung auf, die dazu ausgebildet ist, einen Druck zu erfassen, mit dem das Fluid durch den Fluidpfad der Vorrichtung befördert wird.

Alternativ weist das System eine Durchflussüberwachungseinrichtung auf, die dazu ausgebildet ist, zu überprüfen, ob das Fluid durch den Fluidpfad der Vorrichtung befördert wird. Die Druckerfassungseinrichtung bzw. Durchflussüberwachungseinrichtung sind vorzugsweise in einer der Vorrichtung vorgeschalteten Fluidleitung, bevorzugt Wasserleitung, insbesondere Trinkwasserleitung, angeordnet.

Bezüglich weiterer Merkmale und Vorteile des Systems, insbesondere der Vorrichtung und/oder Druckerfassungseinrichtung und/oder Durchflussüberwachungseinrichtung, wird vollumfänglich auf die im Rahmen des ersten Erfindungsaspekts gemachten Ausführungen Bezug genommen. Die dort insbesondere in Bezug auf die Vorrichtung und/oder Druckerfassungseinrichtung und/oder Durchflussüberwachungseinrichtung beschriebenen Merkmale und Vorteile gelten sinngemäß auch für das System gemäß zweitem Erfindungsaspekt.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt schematisch eine Explosivdarstellung einer Ausführungsform einer erfindungsgemäßen Vorrichtung und
- Fig. 2a,b: zeigen schematisch Ausführungsformen eines erfindungsgemäßen Systems.

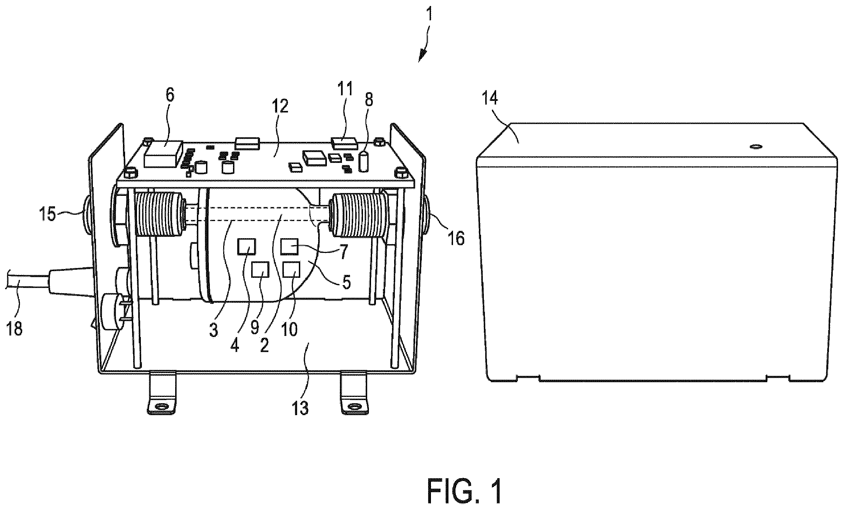

Eine erfindungsgemäße Vorrichtung 1 nach Fig. 1 ist zur Desinfektion eines Fluids 2, vorzugsweise von Trinkwasser, beispielsweise in Reisemobilen, Expeditionsfahrzeugen, Foodtrucks oder Yachten, vorgesehen.

Die Vorrichtung 1 weist einen Fluidpfad 3, durch den das zu desinfizierende Fluid 2 befördert wird, und eine Strahlungsquelle 4 zur Erzeugung einer desinfizierend wirkenden Strahlung auf. Die Strahlungsquelle 4 ist in einem Reaktorgehäuse 5 relativ zu dem Fluidpfad 3 derart angeordnet, dass das Fluid 2 mit der Strahlung während des Förderns (des Fluids 2) beaufschlagt wird.

Die Strahlungsquelle 4 weist wenigstens eine UV-C-LED auf. Zum Beispiel kann die Strahlungsquelle 4 vier UV-C-LEDs aufweisen. Vorzugsweise ist die wenigstens eine UV-C-LED dazu ausgebildet, UV-Strahlung mit einer Wellenlänge von 240 nm bis 290 nm zu erzeugen, wenn die wenigstens eine UV-C-LED aktiv ist. Hierdurch ist mit besonderem Vorteil beispielsweise eine besonders effiziente Trinkwasserentkeimung erzielbar.

Ferner umfasst die Vorrichtung 1 vorzugsweise eine Steuereinheit 6, die dazu ausgebildet ist, einen Betrieb der Vorrichtung 1 zu steuern. Die Vorrichtung 1 weist bevorzugt einen mit der Steuereinheit 6 daten- oder wirkverbundenen Pumpenzustandseingang auf. Dieser ist mit einem Signal, vorzugsweise elektrischen Signal, beispielsweise einer Spannung, beaufschlagbar. Das Signal zeigt an, ob eine Pumpe, beispielsweise eine Druck- oder Tauchpumpe, ein Fördern oder Befördern des Fluids bewirkt. Die Steuereinheit 6 ist dazu ausgebildet, die Strahlungsquelle 4, insbesondere die wenigstens eine UV-C-LED, zu aktivieren, wenn das Signal anzeigt, dass die Pumpe ein Fördern oder Befördern des Fluids 2 bewirkt. Dadurch ist mit besonderem Vorteil eine UV-Desinfektion des Fluids 2 in Echtzeit realisierbar, wodurch die Lebensdauer der Strahlungsquelle 4, insbesondere der wenigstens einen UV-C-LED, signifikant erhöht werden kann.

Ferner kann die Vorrichtung 1 eine Temperaturüberwachungseinrichtung 7 aufweisen, die dazu ausgebildet ist, eine Temperatur der Strahlungsquelle 4, insbesondere der wenigstens einen UV-C-LED, zu überwachen, bevorzugt während die Strahlungsquelle 4, insbesondere die wenigstens eine UV-C-LED, aktiv ist.. Vorzugsweise ist die Temperaturüberwachungseinrichtung 7 ferner dazu ausgebildet, die Auslösung eines Alarm- oder Warnsignals, insbesondere akustischen und/oder optischen Alarm- oder Warnsignals, und/oder die Abschaltung der Vorrichtung 1 zu bewirken, wenn die Temperatur der Strahlungsquelle 4, insbesondere der wenigstens einen UV-C-LED, einen Temperaturschwellenwert, beispielsweise von 52 °C, 53 °C, 54 °C, 55 °C, 56 °C oder 57 °C, überschreitet. Dadurch kann vorteilhafterweise verhindert werden, dass die Strahlungsquelle, insbesondere die wenigstens eine UV-C-LED, überhitzt wird. Dies wiederum führt ebenfalls zu einer Erhöhung der Lebensdauer der Strahlungsquelle 4, insbesondere der wenigstens einen UV-C-LED. Hierzu kann die Vorrichtung 1 eine mit der Temperaturüberwachungseinrichtung 7 daten- oder wirkverbundene Signalgebungseinrichtung 8, beispielsweise in Form einer Signalhupe, aufweisen. Alternativ oder in Kombination kann die Temperaturüberwachungseinrichtung 7 die Abschaltung der Vorrichtung 1 bewirken, wenn die Temperatur der Strahlungsquelle 4, insbesondere der wenigstens einen UV-C-LED, einen der vorgenannten Temperaturschwellenwerte überschreitet.

Ferner kann die Vorrichtung 1 eine Strahlungsintensitätserfassungseinrichtung 9 aufweisen, die dazu ausgebildet ist, eine Intensität der Strahlung, insbesondere UV-Strahlung, zu erfassen, während die Strahlungsquelle 4, insbesondere die wenigstens eine UV-C-LED, aktiv ist. Dadurch ist mit besonderem Vorteil eine Erfassung, insbesondere Echtzeiterfassung, der Desinfektionsleistung der Strahlungsquelle 4, insbesondere der wenigstens einen UC-C-LED, erzielbar. Die Strahlungsintensitätserfassungseinrichtung 9 ist ferner vorzugsweise dazu ausgebildet, die Auslösung eines Alarm- oder Warnsignals zu bewirken, wenn die Intensität der Strahlung, insbesondere UV-Strahlung, einen Strahlungsintensitätsschwellenwert unterschreitet. Hierzu kann die Strahlungsintensitätserfassungseinrichtung 9 ebenfalls mit der Signalgebungseinrichtung 8 daten- oder wirkverbunden sein. Hierdurch kann mit besonderem Vorteil gewährleistet werden, dass das zu desinfizierende Fluid 2 während des Betriebs der Vorrichtung 1 mit einer ausreichend desinfizierend wirkenden Strahlung, insbesondere UV-Strahlung, beaufschlagt wird. Alternativ oder in Kombination kann die Strahlungsintensitätserfassungseinrichtung 9 die Abschaltung der Vorrichtung 1 bewirken, wenn die Intensität der Strahlung, insbesondere UV-Strahlung, den vorgenannten Strahlungsintensitätsschwellenwert unterschreitet.

Ferner kann die Vorrichtung 1 eine Funktionsüberwachungseinrichtung 10 aufweisen, welche dazu ausgebildet ist, eine Funktion der Strahlungsquelle 4, insbesondere der wenigstens einen UV-C-LED, zu überwachen. Vorzugsweise ist die Funktionsüberwachungseinrichtung 10 dazu ausgebildet, eine Stromversorgung der Strahlungsquelle 4, insbesondere der wenigstens einen UV-C-LED, zu überwachen, während die Strahlungsquelle 4, insbesondere die wenigstens eine UV-C-LED, aktiv ist. Hierdurch kann in vorteilhafter Weise gewährleistet werden, dass die Strahlungsquelle 4, insbesondere die wenigstens eine UV-C-LED, während des Betriebs der Vorrichtung 1 funktioniert, d. h. in der Lage ist, desinfizierend wirkende Strahlung zu erzeugen. Vorzugsweise ist die Funktionsüberwachungseinrichtung 10 ferner dazu ausgebildet, die Auslösung eines Alarm- oder Warnsignals zu bewirken, wenn eine Spannung, die beim/im Betrieb der Vorrichtung 1 die Strahlungsquelle 4, insbesondere die wenigstens eine UV-C-LED, versorgt, einen unteren Spannungsschwellenwert, beispielsweise von 0,17 V, unterschreitet und/oder einen oberen Spannungsschwellenwert, beispielsweise von 0,23 V, überschreitet. Hierdurch kann mit besonderem Vorteil gewährleistet werden, dass die Strahlungsquelle 4, insbesondere die wenigstens eine UV-C-LED, während des Betriebs der Vorrichtung 1 ausreichend mit Strom versorgt ist. Dies wirkt sich ebenfalls positiv auf die Lebensdauer der Strahlungsquelle 4 aus.

Ferner kann die Vorrichtung 1 eine Betriebsüberwachungseinrichtung 11 aufweisen, die dazu ausgebildet ist, zu überprüfen, ob die Vorrichtung 1 mit einer Betriebsspannung versorgt ist. Bevorzugt weist die Vorrichtung 1 ferner einen mit der Betriebsüberwachungseinrichtung 11 datenverbundenen oder wirkverbundenen Pumpenzustands-Eingang auf, der mit einem Signal beaufschlagbar ist, das anzeigt, ob eine Pumpe ein Fördern oder Befördern des Fluids 2 bewirkt. Vorzugsweise ist Betriebsüberwachungseinrichtung 11 dazu ausgebildet, die Auslösung eines Alarm- oder Warnsignals, insbesondere akustischen und/oder optischen Alarm- oder Warnsignals, zu bewirken, wenn das Signal anzeigt, dass die Pumpe ein Fördern oder Befördern des Fluids 2 bewirkt, und die Vorrichtung 1 nicht mit einer Betriebsspannung versorgt ist. Hierzu kann die Betriebsüberwachungseinrichtung 11 ebenfalls mit der Signalgebungseinrichtung 8 daten- oder wirkverbunden sein. Dadurch kann vorteilhafterweise ein Fördern oder Befördern des Fluids 2 durch den Fluidpfad 3 der Vorrichtung 1 verhindert werden, ohne dass gleichzeitig eine Desinfektion des Fluids 2 erfolgt.

Die Steuereinheit 6 und/oder die Signalgebungseinrichtung 8 und/oder die Betriebsüberwachungseinrichtung 11 sind vorzugsweise auf einer Platine 12 angeordnet oder montiert.

Die Temperaturüberwachungseinrichtung 7 und/oder die Strahlungsintensitätserfassungseinrichtung 9 und/oder die Funktionsüberwachung 10 sind vorzugsweise im Reaktorgehäuse 5 angeordnet oder montiert.
Die Vorrichtung weist ferner ein Gehäuse, insbesondere aus Edelstahl, mit einem Gehäuseunterteil 13 und einem Gehäuseoberteil 14 auf.

Die vorgenannten Bauteile/Komponenten der Vorrichtung 1 sind in dem Gehäuse aufgenommen oder eingehaust.

Die Vorrichtung 1 weist ferner einen Fluideintrittsanschluss 15 sowie einen Fluidaustrittsanschluss 16 auf, die jeweils dazu ausgebildet sind, die Vorrichtung 1 mit einer geeigneten Fluidleitung 17, beispielsweise einem Wasserschlauch, zu verbinden. Dadurch ist ein Fördern des zu desinfizierendes Fluids 2 durch den Fluidpfad 3 sowie das Reaktorgehäuse 5, insbesondere von dem Fluideintrittsanschluss 15 in Richtung Fluidaustrittsanschluss 16, erzielbar.

Ferner weist die Vorrichtung 1 ein Geräteanschlusskabel 18 auf, mit Hilfe dessen die Vorrichtung 1 mit einer Betriebsspannung versorgt werden kann.

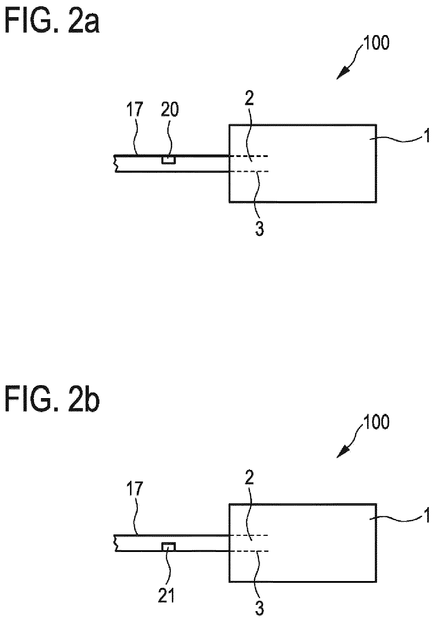

Die Fig. 2a und 2b zeigen jeweils schematisch ein System 100 zur Desinfektion eines Fluids 2, vorzugsweise von Wasser, insbesondere Trinkwasser.

Das System 100 weist eine erfindungsgemäße Vorrichtung 1 auf.

Das System weist ferner eine Druckerfassungseinrichtung 20 auf, die dazu ausgebildet ist, einen Druck zu erfassen, mit dem das Fluid 2 durch den Fluidpfad 3 der Vorrichtung 1 befördert wird (siehe Fig. 2a).

Alternativ weist das System ferner eine Durchflussüberwachungseinrichtung 21 auf, die dazu ausgebildet ist, zu überprüfen, ob das Fluid 2 durch den Fluidpfad 3 der Vorrichtung 1 befördert wird, auf (siehe Fig. 2b).

Die Druckerfassungseinrichtung 20 bzw. Durchflussüberwachungseinrichtung 21 sind vorzugsweise in einer der Vorrichtung 1 vorgeschalteten Fluidleitung 17, bevorzugt Wasserleitung, insbesondere Trinkwasserleitung, angeordnet.

Die Druckerfassungseinrichtung 20 bzw. Durchflussüberwachungseinrichtung 21 haben/hat den Vorteil, dass eine Desinfektion des Fluids 2 nur beim Öffnen einer Armatur, insbesondere Wasserarmatur, stattfindet.

Bezüglich weiterer Merkmale und Vorteile des Systems 100, insbesondere der Vorrichtung 1 und/oder Druckerfassungseinrichtung 20 und/oder Durchflussüberwachungseinrichtung 21, wird vollumfänglich auf die zur Figur 1 gemachten Ausführungen Bezug genommen. Die dort in Bezug auf die Vorrichtung 1 beschriebenen Merkmale und Vorteile gelten sinngemäß.

## Patentansprüche

1. Vorrichtung (1) zur Desinfektion eines Fluids (2), vorzugsweise von Wasser, aufweisend:
- einen Fluidpfad (3), durch den das zu desinfizierende Fluid (2) befördert wird, und
- eine Strahlungsquelle (4) zur Erzeugung von Strahlung, die desinfizierend wirkt, wobei die Strahlungsquelle (4) relativ zum Fluidpfad (3) derart angeordnet ist, dass das Fluid (2) mit der Strahlung während des Förderns beaufschlagt wird,
**dadurch gekennzeichnet, dass**
- die Strahlungsquelle (4) wenigstens eine UV-LED aufweist.

2. Vorrichtung (1) nach Anspruch 1, **dadurch gekennzeichnet, dass**
- die wenigstens eine UV-LED als wenigstens eine UV-C-LED gestaltet ist.

3. Vorrichtung (1) nach Anspruch 1 oder 2, **dadurch gekennzeichnet, dass**
- die wenigstens eine UV-LED dazu ausgebildet ist, UV-Strahlung mit einer Wellenlänge von 240 nm bis 290 nm zu erzeugen.

4. Vorrichtung (1) nach einem der vorhergehenden Ansprüche, **dadurch gekennzeichnet, dass**
- der Fluidpfad (2) abschnittsweise durch ein Reaktorgehäuse (5) geführt ist,
- wobei die Strahlungsquelle (4) in dem Reaktorgehäuse (5) angeordnet ist.

5. Vorrichtung (1) nach einem der vorhergehenden Ansprüche, **dadurch gekennzeichnet, dass**
- die Vorrichtung (1) ferner eine Steuereinheit (6) aufweist, die dazu ausgebildet ist, einen Betrieb der Vorrichtung (1) zu steuern,
- die Vorrichtung (1) einen mit der Steuereinheit (6) wirkverbundenen Pumpenzustands-Eingang aufweist, der mit einem Signal beaufschlagbar ist, das anzeigt, ob eine Pumpe ein Fördern des Fluids (2) bewirkt,
- wobei die Steuereinheit (6) dazu ausgebildet ist, die Strahlungsquelle (4) zu aktivieren, wenn das Signal anzeigt, dass die Pumpe ein Befördern des Fluids (2) bewirkt.

6. Vorrichtung (1) nach einem der vorhergehenden Ansprüche, **dadurch gekennzeichnet, dass**
- die Vorrichtung (1) ferner eine Temperaturüberwachungseinrichtung (7) aufweist, die dazu ausgebildet ist, eine Temperatur der Strahlungsquelle (4) zu überwachen, während die Strahlungsquelle (4) aktiv ist.

7. Vorrichtung (1) nach Anspruch 6, **dadurch gekennzeichnet, dass** die Temperaturüberwachungseinrichtung (7) ferner dazu ausgebildet ist, die Auslösung eines Alarm- oder Warnsignals und/oder die Abschaltung der Vorrichtung (1) zu bewirken, wenn die Temperatur der Strahlungsquelle (4) einen Temperaturschwellenwert überschreitet.

8. Vorrichtung (1) nach einem der vorhergehenden Ansprüche, **dadurch gekennzeichnet, dass**
- die Vorrichtung (1) ferner eine Strahlungsintensitätserfassungsreinrichtung (9) aufweist, die dazu ausgebildet ist, eine Intensität der Strahlung zu erfassen, während die Strahlungsquelle (4) aktiv ist.

9. Vorrichtung (1) nach Anspruch 8, **dadurch gekennzeichnet, dass**
- die Strahlungsintensitätserfassungsreinrichtung (9) ferner dazu ausgebildet ist, die Auslösung eines Alarm- oder Warnsignals und/oder die Abschaltung der Vorrichtung (1) zu bewirken, wenn die Intensität der Strahlung einen Intensitätsschwellenwert unterschreitet.

10. Vorrichtung (1) nach einem der vorhergehenden Ansprüche, **dadurch gekennzeichnet, dass**
- die Vorrichtung (1) ferner eine Funktionsüberwachungseinrichtung (10) aufweist, die dazu ausgebildet ist, eine Funktion der Strahlungsquelle (4) zu überwachen, während die Strahlungsquelle (4) aktiv ist.

11. Vorrichtung (1) nach Anspruch 10, **dadurch gekennzeichnet, dass**
- die Funktionsüberwachungseinrichtung (10) dazu ausgebildet ist, eine Stromversorgung der Strahlungsquelle (4) zu überwachen, während die Strahlungsquelle (4) aktiv ist.

12. Vorrichtung (1) nach einem der vorhergehenden Ansprüche, **dadurch gekennzeichnet, dass**
- die Vorrichtung (1) ferner eine Betriebsüberwachungseinrichtung (11) aufweist, die dazu ausgebildet ist, zu überprüfen, ob die Vorrichtung (1) mit einer Betriebsspannung versorgt ist,
- die Vorrichtung (1) ferner einen mit der Betriebsüberwachungseinrichtung (11) wirkverbundenen Pumpenzustands-Eingang aufweist, der mit einem Signal beaufschlagbar ist, das anzeigt, ob eine Pumpe ein Befördern des Fluids (2) bewirkt,
- wobei die Betriebsüberwachungseinrichtung (11) dazu ausgebildet ist, die Auslösung eines Alarm- oder Warnsignals zu bewirken, wenn das Signal anzeigt, dass die Pumpe ein Befördern des Fluids (2) bewirkt, und die Vorrichtung (1) nicht mit einer Betriebsspannung versorgt ist.

13. Vorrichtung (1) nach einem der vorhergehenden Ansprüche, **dadurch gekennzeichnet, dass**
- die Vorrichtung (1) ferner eine Druckerfassungseinrichtung (20) aufweist, die dazu ausgebildet ist, einen Druck zu erfassen, mit dem das Fluid (2) durch den Fluidpfad (3) befördert wird.

14. Vorrichtung (1) nach einem der vorhergehenden Ansprüche, **dadurch gekennzeichnet, dass**
- die Vorrichtung (1) ferner eine Durchflussüberwachungseinrichtung (21) aufweist, die dazu ausgebildet ist, zu überprüfen, ob das Fluid (2) durch den Fluidpfad (3) befördert wird.

15. System (100) zur Desinfektion eines Fluids (2), vorzugsweise von Wasser, aufweisend
- eine Vorrichtung (1) nach einem der vorhergehenden Ansprüche und
- eine Druckerfassungseinrichtung (20), die dazu ausgebildet ist, einen Druck zu erfassen, mit dem das Fluid (2) durch den Fluidpfad (3) befördert wird, oder
- eine Durchflussüberwachungseinrichtung (21), die dazu ausgebildet ist, zu überprüfen, ob das Fluid (2) durch den Fluidpfad (3) befördert wird.
